(19) Europäisches Patentamt
European Patent Office
Office européen des brevets

(11) EP 0 708 092 A1

# (12) EUROPÄISCHE PATENTANMELDUNG

(43) Veröffentlichungstag:
**24.04.1996 Patentblatt 1996/17**

(51) Int. Cl.[6]: **C07D 213/73**, C07D 471/14, C07D 471/04, A61K 31/44

(21) Anmeldenummer: **94116308.1**

(22) Anmeldetag: **17.10.1994**

(84) Benannte Vertragsstaaten:
**AT BE CH DE DK ES FR GB GR IE IT LI LU NL PT SE**

(71) Anmelder: **MERCK PATENT GmbH**
**D-64293 Darmstadt (DE)**

(72) Erfinder:
- **Fechtel, Ulrich, Dr.**
  **D-64372 Ober-Ramstadt (DE)**
- **Wembacher, Karlheinz**
  **D-64319 Pfungstadt (DE)**
- **Bokel, Heinz-Hermann, Dr.**
  **D-64997 Darmstadt (DE)**

## (54) Verfahren zur Herstellung von 3-Aminopyridinen aus 3-Nitropyridinen

(57) Die Erfindung betrifft ein Verfahren zur Herstellung von 3-Aminopyridinen aus 3-Nitropyridinen, bei dem man die Nitropyridine in saurer Lösung elektrochemisch reduziert.

EP 0 708 092 A1

**Beschreibung**

Diese Erfindung betrifft ein Verfahren zur Herstellung von 3-Aminopyridinen aus 3-Nitropyridinen, bei dem man die Nitropyridine in saurer Lösung elektrochemisch reduziert.

Nach dem neuen Verfahren werden die 3-Nitropyridine der allgemeinen Formel II unter Reduktion der Nitrogruppen und gleichzeitiger Einführung einer Gruppe Y in die 3-Amino-Y-pyridine der allgemeinen Formel I übergeführt; dabei tritt die Gruppe Y ausschließlich in die 2-Stellung ein:

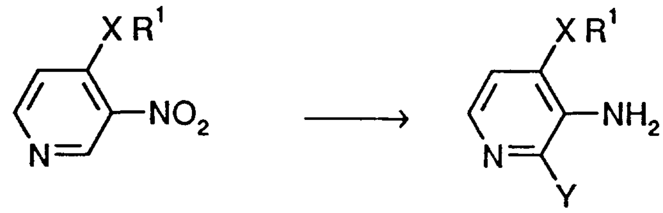

II I

Die 3-Aminopyridine der Formel I werden zur Herstellung von Pharmazeutika, insbesondere von Pyridino [3, 4b] azolen, die als Antikonsulsiva Verwendung finden, z.B. DE 31 50 486 oder DE 41 10 019 oder von Dihydro dipyridoazepinen, die als HIV-Viruzide eingesetzt werden können, z.B. EP 0 429 989, verwendet.

Es ist bekannt, 2-Chlor-3,4-diaminopyridin durch Reduktion von 4-Amino-3-nitropyridin mit (Zinn(II)-chlorid herzustellen (z.B. Bremer, Liebigs Ann. Chem. 518 (1934), 274-280). Der Nachteil dieses Verfahrens liegt in erster Linie im hohen Anfall von Zinn(IV)-Salzen als Abfallstoff, die sowohl die Aufarbeitung des Produktes stark beeinträchtigen als auch zu einer erheblichen Belastung bezüglich ihrer Entsorgung führen.

Bekanntlich lassen sich Nitrobenzole nach der sogenannten "Gattermann-Reaktion" in schwefelsaurer Lösung elektrochemisch unter gleichzeitiger Einführung einer Hydroxygruppe in die entsprechenden Aminophenole überführen. Es gibtjedoch keinerlei Hinweis, daß sich diese elektrochemische Reduktion auch auf Nitropyridine anwenden läßt.

Es wurde nun gefunden, daß man 2-substituierte 3-Aminopyridine vorteilhaft dadurch herstellen kann, daß man 3-Nitropyridine in einer Lösung, die eine Säure und gegebenenfalls ein Salz oder einen Alkohol enthält, elektrochemisch reduziert.

Die Erfindung betrifft demnach ein Verfahren zur Herstellung von 2-Y-3-Aminopyridinen aus den entsprechenden 3-Nitropyridinen unter Einführung der Gruppe Y, wobei man das 3-Nitropyridin einer elektrolytischen Reduktion in Gegenwart einer Säure und gegebenenfalls eines Salzes der Formel III unterzieht,

$$MY \qquad (III)$$

worin

Y Halogen, $OR^1$, $OCOR^1$, $SR^1$, -SCN oder -CN und

M H, Li, Na, K, $NR^2_4$ oder $SiR^2_3$ bedeuten und

$R^1$ und $R^2$ jeweils unabhängig voneinander H, alkyl, Aryl oder Cycloalkyl

bedeuten.

Die Erfindung betrifft insbesondere ein Verfahren zur Herstellung von 2,4-disubstituierten 3-Aminopyridinderivaten der Formel I

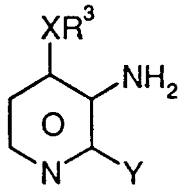

(I)

wobei

| | |
|---|---|
| X | $NR^4$, O, S oder $CH_2$ |
| Y | die angegebene Bedeutung besitzt, und |
| $R^3$ und $R^4$ | jeweils unabhängig voneinander H, Alkyl, Aryl oder Cycloalkyl |

bedeuten,
aus dem entsprechenden 4-substituierten 3-Nitropyridin der Formel II

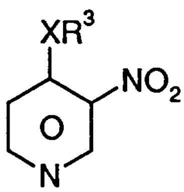

(II)

worin X und $R^3$ die angegebene Bedeutung besitzen, insbesondere ein Verfahren zur Herstellung der Verbindung der Formel Ia

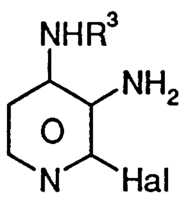

(Ia)

worin $R^3$ die angegebene Bedeutung besitzt, und

Hal Halogen bedeutet.

Bevorzugte Ausführungsformen des erfindungsgemäßen Verfahren sind:

a) Verfahren wobei die Elektrolyse bei Temperaturen zwischen 0 °C und Siedetemperatur des eingesetzten Lösungsmittels durchgeführt wird.

b) Verfahren wobei die Elektrolyse potentiostatisch oder galvanostatisch bei einer Stromdichte zwischen 0,1 und 15 A/dm², vorzugsweise zwischen 1 und 6 A/dm², durchgeführt wird.

c) Verfahren wobei bei der Elektrolyse Kathoden aus Kupfer, Graphit oder Platin verwendet werden.

d) Verfahren wobei die Anodenlösung von der Kathodenlösung durch ein Diaphragma, vorzugsweise ein Diaphragma bestehend aus einer Kationenaustauschermembran mit einem Fluorcarbongrundgerüst getrennt ist.

e) Verfahren wobei die Kathodenlösung 1 bis 20 Gew.% an Verbindung der Formel II und I bis 80 Gew.% an Säure und gegebenenfalls eines Alkohols oder eines Salzes enthält.

Ein weiterer Gegenstand der Erfindung ist ein Verfahren zur Herstellung eines Pyridino[3,4b]azols der Formel IV

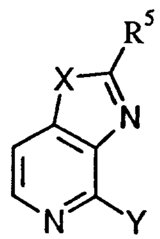

(IV)

worin Y die angegebene Bedeutung besitzt, $R^5$ H oder Alkyl bedeutet, und X' $NR^3$, O, S oder $CHR^3$ bedeutet, dadurch gekennzeichnet, daß man ein 2,4-disubstituiertes 3-Aminopyridin der Formel I nach mindestens einem der Ansprüche 2-7 herstellt und in an sich bekannter Weise mit einer Carbonsäure der Formel $R^5$-COOH oder einem ihrer reaktionsfähigen Derivate umsetzt.

Als 3-Nitropyridinderivate kommt vorzugsweise unsubstituiertes 3-Nitropyridin oder in 4-Stellung substituiertes 3-Nitropyridin, insbesondere 4-Amino- oder 4-Alhexylamino-3-nitropyridin, in Betracht.

Die Art der Alkyl-, Aryl- oder Cycloalkylgruppe in den Resten $R^1$, $R^2$, $R^3$, $R^4$ und $R^5$ ist für die Durchführung des erfindungsgemäßen Verfahrens unkritisch.

Als Alkylgruppen kommen vorzugsweise Alkylgruppen mit 1 bis 10, insbesondere 1 bis 4 C-Atomen in Betracht. Die Alkylreste in den Alkylaminogruppen, Alkoxygruppen und Alkylestergruppen haben vorzugsweise 1 bis 10, insbesondere 1 bis 3 C-Atomen.

Als Arylreste der genannten Art kommen homo- und 5- oder 6-gliedrige heteroaromatische Systeme in Betracht, insbesondere Phenyl-, Pyridin- und Pyrol-Derivate. Bevorzugt sind solche unsubstituierte oder substituierte Phenylreste, die am Benzolring 1 bis 2 Methyl-, Äthyl- oder iso-Propylreste, 1 bis 2 Methoxy- oder Äthoxyreste, 1 bis 2 Fluor-, Chlor- oder Bromatome, 1 bis 2 Carboxylgruppen, 1 Sulfonsäure-, Formyl-, Amino-, Hydroxyl- oder Nitrilgruppe enthalten können. Als Cycloalkylreste kommen vorzugsweise Cycloalkylgruppen mit 5 bis 8, vorzugsweise 6 C-Atomen in Betracht.

Die saure Lösung der 2-Nitropyridinverbindung enthält als Säure z.B. Salzsäure, Schwefelsäure oder Methylschwefelsäure. Sie kann außerdem einen Elektrolyten, wie Tetrabutylammoniumhydrogensulfat und ein Kolösungsmittel, wie Wasser oder Dioxan enthalten. Als Säure kommt bevorzugt Salzsäure oder Schwefelsäure in Betracht. Die als Katholyt zu verwendende Lösung der Nitropyridinverbindung enthält vorzugsweise bis zu 80 Gewichtsprozent Säure. Die Konzentration der Nitroverbindung im Katholyten kann zwischen1 und 20 Gewichtsprozent liegen. Beispielsweise setzt sich der Katholyt zusammen aus 1 bis 20 Gewichtsprozent der Nitroverbindung, 20 bis 40 Gewichtsprozent Chlorwasserstoff und 40 bis 80 Gewichtsprozent Wasser.

Die erfindungsgemäße Elektrosynthese kann in einer üblichen Elektrolysezelle durchgeführt werden, bei der Anoden- und Kathodenraum durch ein Diaphragma, z.B. aus porösem Ton oder einer handelsüblichen lonenaustauschermembran, getrennt sind, wie in einer Zelle vom Filterpressentyp, insbesondere einer Kationenaustauschermembran mit einem Fluorcarbongrundgerüst vom Nafion®-Typ.

Die Kathode besteht z.B. aus einer Platte aus Pb, Sn, Zn, $V_2A$, Fe, Cu, Ni, Hg, Au, Ag, Pt, Pd, Ti, Al oder aus Legierungen dieser Metalle. Sie kann auch aus Graphit oder graphitgefülltem Kunststoff oder aus einem Quecksilbersee bestehen und in Form von Streckmetallen, Kugeln, Geweben oder Filzen angewendet werden.

Die Anode kann aus einer Graphit- oder $Pb/PbO_2$-Elektrode oder aus aktivierten und/oder beschichteten Streckmetallen, z.B. Titan, bestehen. Es können auch andere Anoden, wie sie in der organischen Elektrochemie bekannt sind, eingesetzt werden. Der Anolyt hat die an sich übliche Zusammensetzung. Er besteht vorzugsweise aus wäßriger Mineralsäure, vorzugsweise Salzsäure oder Schwefelsäure.

Man führt die elektrochemische Reduktion bei Temperaturen bis zum Siedepunkt des Lösungsmittels, vorzugsweise bis 90 °C, insbesondere zwischen 50 und 60 °C, durch. Man kann den Katholyten z.B. durch Beheizen der Kathode, des Kathoden- oder Anodenkreislaufs oder durch die Reaktionswärme auf die gewünschte Temperatur einstellen. Eine

Kühlung ist entsprechend möglich. Der Katholyt wird zweckmäßig gerührt oder umgepumpt. Beim Anolyten genügt oft die Zirkulation durch das Anodenabgas.

Man führt die Elektrolyse z.B. so durch, daß man den Umsatz der Nitroverbindung polarographisch verfolgt. Das entstehende Aminoprodukt und seine Nebenprodukte können titriert werden.

Ein Rühren auf der Kathode wie ein zügiges Vorbeiströmen des Katholyten an der Kathode ist vorteilhaft.

Die Elektrolyse kann sowohl potentiostatisch als auch galvanostatisch durchgeführt werden.

Die Stromdichte ist bei dem erfindungsgemäßen Verfahren nicht kritisch, sie liegt z.B. bei 0,1 bis 50 $A/dm^2$, vorzugsweise bei 1,0 bis 15 $A/dm^2$, insbesondere zwischen 1 und 6 $A/dm^2$.

Nach dem erfindungsgemäßen Verfahren lassen sich wertvolle Aminopyridine, die bisher nur auf umständliche Weise hergestellt werden konnten, besonders einfach und vorteilhaft herstellen. So wird z.B. das für Pharmazeutika benötigte Zwischenprodukt 3,4-Diamino-2-chlorpyridin auf herkömmliche Weise durch Reduktion von 4-Amino-3-nitropyridin mit Zinn(II)-chlorid hergestellt.

Das vorteilhafte Ergebnis des erfindungsgemäßen Verfahrens, das u.a. auch darin besteht, daß keine Zinn(IV)-salze entsorgt werden müssen, muß aufgrund des Standes der Technik als überraschend bezeichnet werden.

<u>Beispiel 1</u>

3,4-Diamino-2-chlorpyridin aus 4-Amino-3-nitropyridin

| | |
|---|---|
| Elektroden: | Graphit |
| Diaphragma: | Nafion® 417 |
| Anolyt: | 37%ige HCl 1 L |
| Katholyt: | 1,4 L 37%ige HCl - 17,5 g 4-Amino-3-nitro-pyridin |

Die Elektrolyse wurde in einer Durchflußzelle bei ca. 60 °C durchgeführt. Es wurde galvanostatisch mit einer Stromdichte von 1,5 $A/dm^2$ bis zu 128 % der erforderlichen Ladungsmenge elektrolysiert. Nach dem Abbruch der Elektrolyse wurde der Katholyt entnommen und das Dihydrochlorid des 2-Chlor-3,4-diamino-pyridins durch Abkühlung im Eisbad und Stehen über Nacht auskristallisiert. Insgesamt konnten 25,4g Produkt isoliert werden (92 % d.Th.).

<u>Beispiel 2:</u>

3,4-Diamino-2-methoxypyridin aus 4-Amino-3-nitropyridin

In der in Beispiel 1 beschriebenen Apparatur wird eine Lösung aus 400 ml Methanol, 15 ml Schwefelsäure und 17,5 g 4-Amino-3-nitropyridin bei ca. 50 °C und einer Stromdichte von 1,5 $A/dm^2$ bis zu 120 % der erforderlichen Ladungsmenge elektrolysiert.

Anschließend wird das Methanol abdestilliert und der Rückstand mit Wasser versetzt. Nach Extraktion mit Dichlormethan und üblichen Aufarbeiten erhält man das Produkt.

<u>Beispiel 3:</u>

3,4-Diamino-2-hydroxy-pyridin aus 4-Amino-3-nitropyridin

| | |
|---|---|
| Katholyt: | 450 ml 25%ige wäßrige Schwefelsäure 20 g 4-Amino-3-nitro-pyridin |
| Anolyt: | 75 ml 25%ige wäßrige Schwefelsäure |
| Kathode: | Graphit |
| Anode: | Blei |
| Diaphragma: | Keramikfritte |
| Stromdichte: | 50 $mA/cm^2$ |
| Temperatur: | 50 °C |

Die Elektrolyse wurde nach dem Durchgang von 107 % der theoretisch erforderlichen Ladungsmenge abgebrochen. Danach wurde im Elektrolyseprodukt das Zielprodukt mit einem HPLC-Anteil von 86 % gefunden.

<u>Beispiel 4:</u>

3,4-Diamino-2-methoxy-pyridin aus 4-Amino-3-nitropyridin

| | |
|---|---|
| Katholyt: | 450 ml 25%ige methanolische Schwefelsäure 10 g 4-Amino-3-nitro-pyridin |

| | |
|---|---|
| Anolyt: | 75 ml 25%ige methanolische Schwefelsäure |
| Kathode: | Graphit |
| Anode: | Graphit |
| Diaphragma: | Keramikfritte |
| Stromdichte: | 10 mA/cm$^2$ |
| Temperatur: | 40 °C |

Die Elektrolyse wurde nach dem Durchgang von 113 % der theoretisch erforderlichen Ladungsmenge abgebrochen. Im Elektrolyseprodukt wurde das 3,4-Diamino-2-methoxy-pyridin mit einem HPLC-Anteil von 93 % nachgewiesen.

## Patentansprüche

1. Verfahren zur Herstellung von 2-Y-substituierten 3-Aminopyridinderivaten wobei

   Y Halogen, $OR^1$, $OCOR^1$, $SR^1$, -SCN oder CN

   bedeutet,
   aus dem entsprechenden 3-Nitropyridinen unter Einführung einer nucleophilen Gruppe Y, dadurch gekennzeichnet, daß man das 3-Nitropyridinderivat einer elektrolytischen Reduktion in Gegenwart einer Säure und gegebenenfalls eines Alkohols oder eines Salzes der Formel III

   $$MY \qquad (III)$$

   worin

   | | |
   |---|---|
   | Y | die angegebene Bedeutung besitzt, |
   | M | H, Li, Na, K, $NR^2_4$ oder $SiR^2_3$,und |
   | $R^1$ und $R^2$ | unabhängig voneinander H, Alkyl, Aryl oder Cycloalkyl |

   bedeuten,
   unterzieht.

2. Verfahren nach Anspruch 1 zur Herstellung von 2,4-disubstituierten 3-Aminopyridinderivaten der Formel I

   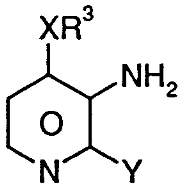

   (I)

   wobei

   | | |
   |---|---|
   | X | $NR^4$, O, S oder $CH_2$ |
   | Y | die angegebene Bedeutung besitzt, |
   | $R^3$ und $R^4$ | jeweils unabhängig voneinander H, Alkyl, Aryl oder Cycloalkyl |

bedeuten,
aus dem entsprechenden 4-substituierten 3-Nitropyridin der Formel II

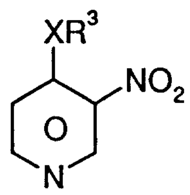

(II)

worin X und $R^3$ die angegebene Bedeutung besitzen.

3. Verfahren nach Anspruch 2 zur Herstellung der Verbindung der Formel Ia

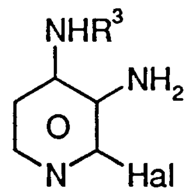

(Ia)

worin $R^3$ die angegebene Bedeutung besitzt, und

Hal Halogen bedeutet.

4. Verfahren nach Anspruch 1, 2 oder 3, dadurch gekennzeichnet, daß die Elektrolyse bei Temperaturen zwischen 0 °C und der Siedetemperatur des eingesetzten Lösungsmittels durchgeführt wird.

5. Verfahren nach einer der vorangehenden Ansprüche, dadurch gekennzeichnet, daß die Elektrolyse galvanostatisch bei einer Stromdichte zwischen 0,1 und 15 A/dm$^2$, vorzugsweise zwischen 2 und 4 A/dm$^2$, durchgeführt wird.

6. Verfahren nach einem der vorangehenden Ansprüche dadurch gekennzeichnet, daß bei der Elektrolyse Kathoden aus Kupfer, Graphit oder Platin verwendet werden.

7. Verfahren nach einem der vorangehenden Ansprüche, dadurch gekennzeichnet, daß die Anodenlösung von der Kathodenlösung durch ein Diaphragma, vorzugsweise ein Diaphragma bestehend aus einer Kationenaustauschermembran mit einem Fluorcarbongrundgerüst getrennt ist.

8. Verfahren nach einem der vorangehenden Ansprüche, dadurch gekennzeichnet, daß die Kathodenlösung 1 bis 20 Gew.% an Verbindung der Formel II und 1 bis 80 Gew.% an Säure und gegebenenfalls eines Alkohols oder eines Salzes enthält.

**9.** Verfahren zur Herstellung eines Pyridino[3,4b]azols der Formel IV

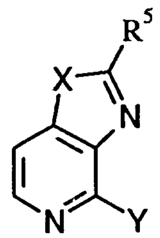

(IV)

worin Y die angegebene Bedeutung besitzt, $R^5$ H oder Alkyl bedeutet, und X' $NR^3$, O, S oder $CHR^3$ bedeutet, dadurch gekennzeichnet, daß man ein 2,4-disubstituiertes 3-Aminopyridin der Formel I nach mindestens einem der Ansprüche 2-7 herstellt und in an sich bekannter Weise mit einer Carbonsäure der Formel $R^5$-COOH oder einem ihrer reaktionsfähigen Derivate umsetzt.

**10.** Verfahren zur Herstellung eines Dihydrodipyridoazepins der Formel V

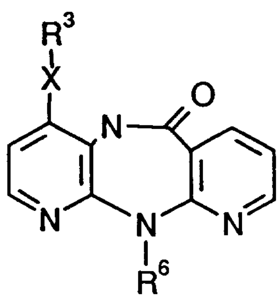

(V)

worin $R^3$ die angegebene Bedeutung besitzt und $R^6$ eine der für $R^3$ angegebenen Bedeutungen aufweist, dadurch gekennzeichnet, daß man ein 4-substituiertes 3-Amino-2-chlorpyridin nach mindestens einem der Ansprüche 2-7 herstellt und in an sich bekannter Weise nacheinander mit 2-Chlornicotinoylchlorid und einem primären Amin umsetzt und das erhaltene Produkt mit einer starken Base behandelt.

Europäisches Patentamt

# EUROPÄISCHER RECHERCHENBERICHT

Nummer der Anmeldung
EP 94 11 6308

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | COLLECTION OF CZECHOSLOVAK CHEMICAL COMMUNICATIONS, Bd.58, Nr.3, 1993, PRAGUE CS Seiten 629 - 648 H. DVORAKOVA ET AL. 'Synthesis and biological effects of acyclic analogs of deazapurine nucleosides.' *Seite 631 und 640* | 1-10 | C07D213/73 C07D471/14 C07D471/04 A61K31/44 |
| A | EP-A-0 598 396 (BOEHRINGER INGELHEIM PHARMACEUTICALS INC.) * das ganze Dokument * | 1-10 | |
| A,D | EP-A-0 429 987 (BOEHRINGER INGELHEIM PHARMACEUTICALS INC.) * das ganze Dokument * | 1-10 | |
| A<br>D | EP-A-0 505 893 (MERCK PATENT GESELLSCHAFT MIT BESCHRÄNKTER HAFTUNG)<br>& DE-A-41 10 019 | 1-10 | |

RECHERCHIERTE SACHGEBIETE (Int.Cl.6)
C07D
A61K

Der vorliegende Recherchenbericht wurde für alle Patentansprüche erstellt

| Recherchenort | Abschlußdatum der Recherche | Prüfer |
|---|---|---|
| DEN HAAG | 16. Dezember 1994 | Bosma, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)